(19) Europäisches Patentamt
European Patent Office
Office européen des brevets

(11) Publication number: **0 369 425 A1**

(12) # EUROPEAN PATENT APPLICATION

(21) Application number: **89121138.5**

(51) Int. Cl.5: **C12P 21/08, C12N 5/24**

(22) Date of filing: **15.11.89**

(30) Priority: **16.11.88 JP 289689/88**

(43) Date of publication of application:
**23.05.90 Bulletin 90/21**

(84) Designated Contracting States:
**AT BE CH DE ES FR GB GR IT LI LU NL SE**

(71) Applicant: **MECT CORPORATION**
**2-1-1, Nishi-Shinjyuku Shinjyuku-ku**
**Tokyo(JP)**

(72) Inventor: **Hino, Akihiro**
**1-11-8, Kotesashi-cho**
**Tokorozawa-shi Saitama-ken(JP)**
Inventor: **Kannagi, Reiji**
**19, Tojiinkitamachi, Kita-ku**
**Kyoto-shi Kyoto-fu(JP)**
Inventor: **Suda, Isao**
**Takadaso, 2886-5, Yayoimachi**
**Tokorozawa-shi Saitama-ken(JP)**
Inventor: **Numata, Masaaki**
**1785, Yamada**
**Kawagoe-shi Saitama-ken(JP)**
Inventor: **Shibayama, Shohei**
**5-goshitsu, 3-52-16 Kitanaka**
**Tokorozawa-shi Saitama-ken(JP)**
Inventor: **Sugimoto, Mamoru**
**1-26-8, Kamitakada Nakano-ku**
**Tokyo(JP)**
Inventor: **Tomita, Kenkichi**
**Meguro-Coporasu 504, 3-24-14,**
**Shimomeguro**
**Meguro-ku Tokyo(JP)**
Inventor: **Ogawa, Tomoya**
**3-6-6-3-101, Kichijyojikitamachi**
**Musashino-shi Tokyo(JP)**

(74) Representative: **Vossius & Partner**
**Siebertstrasse 4 P.O. Box 86 07 67**
**D-8000 München 86(DE)**

(54) **Monoclonal antibody specifically recognizing N-glycolyl type GM2, hybridoma producing the antibody and method for preparing the hybridoma.**

(57) A monoclonal antibody having specificity to N-glycolyl type $GM_2$ and a hybridoma producing a monoclonal antibody which has specificity to N-glycolyl type $GM_2$ are described. The hybridoma can be established according to a method which comprises the fusion of (A) B-cells obtained by immunizing an animal from which thymus is removed when it is a neonate using a synthetic N-glycolyl type $GM_3$ as an immunogen, and (b) myeloma cells. The monoclonal antibody can be used in, for instance, ELISA and TLC immunostaining which are usually adopted for the detection of cancer-related glycolipids.

EP 0 369 425 A1

# Monoclonal Antibody Specifically Recognizing N-Glycolyl Type $GM_2$, Hybridoma Producing the Antibody and Method for Preparing the Hybridoma

The present invention relates to a monoclonal antibody specifically recognizing N-glycolyl type $GM_2$, a novel hybridoma capable of producing such a monoclonal antibody and a method for establishing such a hybridoma.

The glycolipids of mammalian cells belong to the category of so-called sphingoglycolipids and comprise (i) a lipid structure referred to as ceramide composed of a long-chain aminoalcohol called sphingosine to which a fatty acid is acid amido-bonded and (ii) various combinations of sugars selected from the group consisting of glucose, galactose, N-acetylglucosamine, N-acetylgalactosamine and sialic acid, which are bonded to the structure through glycoside bonds.

These compounds are generally located in the outer molecular layer of the two-molecular-layer structure of lipids of cell membrane and it is thought, from recent investigations, that the compounds play an important role in functions such as reception of information for discrimination in the cells and response to such information; receptor functions; differentiation; and proliferation, malignant change, and other behaviors of cells.

Among these glycolipids, the N-glycolyl type $GM_2$ is considered to be one of the H-D antigens and has been detected in sera of patients suffering from a variety of diseases such as rheumatic arthritis and cancer.

The monoclonal antibody has high specificity to a specific antigen and thus can detect the same with high sensitivity. Therefore, if a monoclonal antibody specific to the N-glycolyl type $GM_2$ is obtained, it would be expected that such a monoclonal antibody could be applied to diagnosis (serum diagnosis) of such diseases as mentioned above and to immunological treatment, as a marker for such diseases. Thus, there has been a strong demand for the development of a monoclonal antibody specific to the N-glycolyl type $GM_2$.

Accordingly, a principal object of the present invention is to provide a monoclonal antibody specific to N-glycolyl type $GM_2$.

Another object of the present invention is to provide a novel hybridoma capable of producing such a monoclonal antibody specific to N-glycolyl type $GM_2$.

A further object of the present invention is to provide a method for establishing a hybridoma capable of producing such a monoclonal antibody specific to N-glycolyl type $GM_2$.

According to the present invention it was found that said monoclonal antibody can be obtained by using a synthetic glycolipid, N-glycolyl type $GM_2$ as an immunogen.

According to one aspect of the present ivention, there is provided a monoclonal antibody which has specificity to N-glycolyl type $GM_2$.

According to a second aspect of the present invention, there is provided a novel hybridoma capable of producing a monoclonal antibody specific to N-glycolyl type $GM_2$.

According to a further aspect of the present invention, there is provided a method for establishing a novel hybridoma capable of producing a monoclonal antibody specific to N-glycolyl type $GM_2$ which comprises the step of cell-fusing (A) B cells, which are obtained by immunizing an animal from which the thymus is removed when it is a neonate with synthetic N-glycolyl type $GM_3$ as an immunogen, with (b) myeroma cells.

Fig. 1 is a diagram showing the specificity, to a variety of glycolipids, of the monoclonal antibody according to the present invention determined by the enzyme-labeled antibody technique; and

Figs. 2 and 3 are diagrams showing the specificity of the monoclonal antibody according to the present invention determined by TLC immunostaining technique.

The monoclonal antibody, the hybridoma and the method for establishing the hybridoma according to the present invention will hereunder be explained in more detail.

The hybridoma capable of producing the monoclonal antibody of the present invention can be obtained according to the method of Koehler and Millstein. More specifically, it can be obtained by cell-fusing a B cell (B lymphocyte), which is obtained by immunizing an animal with an antigen, with a myeloma cell. In the present invention, a synthetic glycolipid, N-glycolyl type $GM_3$, is used as such an antigen. The synthetic glycolipid, N-glycolyl type $GM_3$, is a compound represented by the following structural formula:

Gal β1-4 Glc β1-1Cer
3
|
2
NeuGc α

Any kind of animal may be used as the animal immunized with the foregoing antigen, and specific

examples thereof include rabbit, human, mouse and rat, preferably mouse and more preferably BALB/c mouse. BALB/c mice from which the thymus is removed when they are neonates (NTX mice) are preferably used in the present invention. The thymus of the NTX mice is generally removed by a surgical operation several days after birth. The B cells (B lymphocytes) thus obtained are antibody-producing cells and preferred antibody-producing cells are spleen cells. On the other hand, "myeroma cells" used in the present 'invention may also be those derived from rabbit, human, mouse and rat, preferably mouse and more preferably myeroma cells (Sp 2/0) derived from BALB/c mouse. These myeloma cells are fused with the foregoing B cells, in particular spleen cells.

It is known that antibodies are proteins having an ability of inking with and recognizing a molecule known as an antigen. Moreover, the so-called monoclonal antibody is an antibody produced by cloned cells derived from a single cell. Therefore, it recognizes only one kind of antigenic determinant.

Thus, the monoclonal antibody according to the present invention specifically recognizes N-glycolyl type $GM_2$, which is a kind of H-D antigen. The N-glycolyl type $GM_2$ is a sialic acid type sphingoglycolipid having the following chemical structural formula:

```
GalNAcβ1-4Galβ1-4Glcβ1-1Cer
            3
            |
     NeuGc α2
```

There have been known the following sialic acid glycolipids as the glycolipids having a chemical structure similar to that of N-glycolyl type $GM_2$. However, the monoclonal antibody of the present invention does not recognize these compounds at all:

$GM_2$; $GM_3$; N-Gc $GM_3$; $GD_{1a}$; $GD_{1b}$; and $GM_1$

According to the Ouchterlony method, the monoclonal antibody of the present invention belongs to IgM class.

The method for obtaining the hybridoma of the present invention will be explained in detail below.

First of all, the synthetic glycolipid, N-glycolyl type $GM_3$, is injected into an animal, for instance a mouse, intraperitoneally, subcutaneously or intravenously, preferably intraperitoneally, to immunize the mouse. In this immunization, either incomplete Freund's adjuvants or complete Freund's adjuvants may be used as an adjuvant, i.e., an agent for immunological enhancement and specific examples thereof are oils, emulsifying agents, killed tubercule bacillus, killed Salmonella and mixture thereof, preferably killed Salmonella minesota for intraperitoneal or subcutaneous injection. These antigens and adjuvants are preferably used in the form of a solution having an approximately physiologically acceptable composition, such as a solution in phosphate-buffered saline.

Then, B cells (B lymphocytes) are isolated from the aforementioned immunized mice and are fused with myeloma cells.

As an agent for cell fusion, there can be used, for instance, polyethylene glycol and HVJ, polyethylene glycol being preferably.

In addition, it is preferred to use HAT medium as the culture medium so as to enable hybridoma cells to separate easily from myeloma cells which remain unfused.

Thereafter, the resultant hybridoma cells are subjected to cloning operation such as by methyl cellulose technique, soft agarose technique or limiting dilution technique, to isolate a desired single cloned hybridoma capable of producing the intended antibody.

The antibody titer of the monoclonal antibody producing hybridoma thus established can be examined in an ordinary manner to select hybridoma exhibiting high antibody titer. The hybridoma thus selected is then stored.

The monoclonal antibody according to the present invention can be used in, for instance, ELISA and TLC immunostaining which are usually adopted for the detection of cancer-related glycolipids. Therefore, the monoclonal antibody produced by the hybridoma of the present invention can be used for detecting an H-D antigen present in the tissues of human and animals.

Moreover, the monoclonal antibody of the present invention may be used in affinity chromatography for purifying antigens capable of being bonded thereto. In addition, if the monoclonal antibody of the present invention is labeled with a radioisotope, it may be used to detect tumors or to accurately identify the position thereof and it may be used in a high dosage to treat patients suffering from tumor.

It can also be linked to any chemotherapeutic agent to enhance the toxicity thereof to cancer cells.

The monoclonal antibody of the present invention can be used for treating or diagnosing species having the antigen specific to the monoclonal antibody, such as human and animals, for instance mouse.

The present invention will be explained in more detail with reference to the following non-limitative working Examples and the effect practically achieved by the present invention will also be discussed in detail.

Example

(A) Preparation of Samples

(1) Synthesis of N-Glycolyl Type $GM_3$

(Refer to "Carbohydrate Research" 174 (1988), 73 - 85)

(2) Preparation of Antigen Solution

The synthetic glycolipid produced according to the foregoing method (210 µg) and Salmonella minesota R 595 treated with acetic acid (820 µg) as an adjuvant were mixed in hot distilled matter (50 ° C, 5ml) to prepare antigen mixture. This mixture was lyophylized and stored in freezer.

Before use this mixture was dissolved in appropriate volume of phosphate buffered saline (hereunder referred to as "PBS(-)") to prepare an antigen solution.

(3) Test Animals

As test animals, there were used BALB/c mice (NTX mice) whose thymus had been removed when they are neonates, more specifically one-year-old female BALB/c mice whose thymus had been removed by a surgical operation 3 days after birth.

(4) Culture Medium

GIT Medium: A medium available from WAKO JUNYAKU CO., LTD was used.
HAT Medium: 0.0388 g of thymidine and 0.1361 g of hypoxanthine were dissolved in 100 mℓ of distilled water under heating and the resulting solution (a) was stored at -20° C as a stock solution having a concentration 100 times higher than the desired one. Likewise, 0.0176 g of aminopterin was dissolved in 100 mℓ of distilled water by adding a small amount of a 1N sodium hydroxide aqueous solution, and then this was diluted 10 times with GIT culture medium and the resultant solution (b) was stored at -20 ° C as a stock solution having a concentration 100 times higher than the desired one while shielding the light. The desired HAT medium was prepared by adding 1/100 volume each of these two stock solutions (a) and (b) to the GIT culture medium immediately before use.

Moreover, HT medium was prepared by simply adding 1/100 volume of the stock solution (a) containing hypoxanthin and thymidine to the GIT culture medium.

B. Preparation of Hybridoma

(1) Method of Immunization

The aforementioned mice whose thymus had been removed when they were neonates were immunized by intraperitoneally injecting the foregoing immunogen solution in accordance with the following immunization schedule (the amount injected was expressed in terms of the amount of the synthetic glycolipid): initially 5 µg; 10 µg at 4 days after; 15 µg at 7 days after; 20 µg at 11 days after; and 20 µ g at 21 days after. 24 Days after the first immunization, the mice were sacrificed to dissect away the spleen thereof and a suspension of individual spleen cells was prepared therefrom to subsequently use the same in cell fusion.

(2) Method for Cell Fusion

Fusion of the spleen cells obtained above and the mouse myeroma cells, i.e., Sp 2/0, was performed according to the method of Koehler and Millstein. More specifically, 1 X $10^8$ spleen lymphocytes were fused with $10^7$ mouse myeroma cells in the presence of 50% polyethylene glycol (PEG 6000) in the GIT culture medium.

(3) Selection and Breeding of Hybridoma

After the cell fusion, the resultant fused cells were cultured in the HAT culture medium at 37° C in the presence of 5% $CO_2$.

14 Days after the cell fusion, ELISA was performed utilizing the synthetic glycolipid and N-glycolyl type $GM_2$ extracted from mouse liver (NeuGc $GM_2$) to thus obtain only one positive well (A 8).

The cells in the positive well (A 8) were cloned twice according to the limiting dilution technique to thus establish a desired hybridoma. The hybridoma thus established was deposited with American Type Culture Collection (ATCC) under the accession number of HB 9816.

C. Estimation of the Reactivity of the Monoclonal Antibody with the Glycolipid

(1) ELISA

The glycolipid together with phosphatidyl choline and cholesterol were adsorbed on a 96-well flat bottomed plate (available from Flow Laboratories, Inc.) and the plate thus treated was used in the following experiments. 50 $\mu$ ℓ each of a solution containing phosphatidyl choline, cholesterol and the glycolipid in ethanol (the respective components being adjusted to their optimum concentration of 5 $\mu$g/m ℓ, 2.5 $\mu$g/m ℓ and 0.5 $\mu$g/m ℓ ) was pipetted into the wells of the plate, then the solvent was evaporated off therefrom, 200 $\mu$ ℓ each of 5% bovine serum albumin PBS(-) solution was added to the wells and they were allowed to stand at room temperature for 3 hours. The plate was held upside down and shaken to remove the solution, then 200 $\mu$ ℓ each of 0.5% bovine serum albumin PBS(-) solution was added to the wells and the solution was removed in the same manner to wash the wells. The washing treatment was repeated twice. Then, 50 $\mu$ ℓ each of of the supernatant of the hybridoma culture medium, as the primary antibody, was added and the plate was allowed to stand at room temperature for one hour. Likewise, the primary antibody was removed from the wells, and then the wells were washed three times by adding 200 $\mu$ ℓ each of 0.5% PSA-PBS solution. After the removal of this solution, 50 $\mu$ ℓ each of a secondary antibody was added to the wells and 0.5% bovine serum albumin PBS(-) solution was added to each wells. As in the case of the primary antibody, the wells were washed four-times with 0.5% bovine serum albumin PBS(-) solution and 50 $\mu$ ℓ each of the reaction solution was pipetted into the wells to carry out the reaction at room temperature for 10 minutes. The reaction solution used was prepared by dissolving, into citrate-phosphate buffer (pH 5), o-phenylenediamine and hydrogen peroxide so that the final concentrations thereof were 0.4 mg/m ℓ and 0.006%, respectively. The reaction was stopped by the addition of 50 $\mu$ ℓ each of a 2N sulfuric acid solution and then the product was examined by colorimetry at 500 nm. As the secondary antibody, there were herein used the standard goat anti-mouse IgG and M antibodies labeled with horseradish peroxidase (HRP).

The results obtained are plotted in Fig. 1.

In this experiment, there were used NeuGc $GM_2$, NeuGc $GM_3$, NeuAc $GM_2$ and NeuAc $GM_3$ as the glycolipids. NeuGc $GM_2$ was extracted from the liver of BALB/c mice. NeuGc $GM_3$ was synthetized. NeuAc $GM_2$ and NeuAc $GM_3$ were obtained from FUNAKOSHI CO., LTD.

The results observed are also plotted on Fig. 1.

As seen from the results plotted on Fig. 1, the supernatant of the culture medium of A 8 strongly reacted with NeuGc $GM_2$, but it did not react with NeuGc $GM_3$, NeuAc $GM_2$ and NeuAc $GM_3$ having structures similar to that of NeuGc $GM_2$, at all.

Moreover, the reactivity of the supernatant of the culture medium of A 8 was also examined on NeuAc $GM_1$ (FUNAKOSHI), NeuAc $GD_{1a}$ - (FUNAKOSHI), NeuAc $GD_{1b}$ (FUNAKOSHI), CDH (extracted from type O red cell membrane), CTH (extracted from type O red cell membrane), paragloboside (extracted from type O red cell membrane), asialo $GM_1$ (prepared from $GM_1$) and asialo $GM_2$ (prepared from $GM_2$) as antigens according to ELISA. However, the supernatant of the culture medium of A 8 did not react with these glycolipids at all.

It is found, from these results explained above, that the culture medium of A 8 specifically reacts with NeuGc $Gm_2$.

(2) TLC Immunostaining Technique

A silica gel thin layer chromato plate (TLC; HPRC plate available from BAKER Company) was cut into pieces of an appropriate size, each of which was spotted with a small drop of the solution of one glycolipid in chloroform-methanol (2:1 (v/v)). The plate was developed with a developing solution (chloroform-methanol-0.5% CaC $\ell_2$ = 55:45:10 (v/v)) and then was allowed to stand in a 5% bovine serum albumin solution at 4° C over night. After washing the plate with 0.5% bovine serum albumin PBS(-) solution, it was dipped in the supernatant of the culture medium of A 8 as the primary antibody and was reacted wth the primary antibody at room temperature for one hour. After sufficiently washing it with 0.5% bovine serum albumin PBS(-) solution, it was sufficiently dipped in a secondary antibody (rabbit anti-mouse IgM antibody) which was diluted to its optimum concentration with 0.5% bovine serum albumin PBS(-) solution at room temperature for one hour, sufficiently washing it with 0.5% bovine serum albumin PBS(-) solution and then reacting it with $^{125}I$ labeled protein A which was diluted to its optimum concentration with 0.5% bovine serum albumin PBS(-) solution at room temperature for one hour. The plate was washed three times with 0.5% bovine serum albumin PBS(-) solution and additionally two times with PBS(-) solution and then autoradiography was peformed.

The results of qualitative tests and immunoreactions of various kinds of glycolipids obtained by TLC staining technique (orcinol staining) (A) and TLC immunostaining technique (B) are shown in Fig. 2. Fig. 2 shows the results obnserved on NeuGc $GM_2$, NeuAc $GM_2$ and an acidic glycolipid derived from type O red cell membrane. On the other hand, Fig. 3 shows the results observed on a variety of glycolipids inclusive of the foregoing ones.

The glycolipids (see Fig. 2) used in these tests are as follows:

(1) Lane 1: the acidic glycolipid derived from the type O red cell membrane;

(2) Lane 2: NeuGc $GM_2$ 1 µg;

(3) Lane 3: NeuGc $GM_2$ 3 µg;

(4) Lane 4: NeuGc $GM_2$ 10 µg;

(5) Lane 5: NeuAc $GM_2$ 2 µg;

(6) Lane 6: NeuAc $GM_2$ 10 µg;

As seen from the results shown in Fig. 2, the supernatant of the culture medium of A 8 reacts only with NeuGc $GM_2$, but not with the acidic glycolipid derived from the type O red cell membrane or NeuAc $GM_2$.

In addition, the glycolipid spotted on the lane e in tests shown in Fig. 3 is the acidic glycolipid derived from the liver of BALB/c mice whose principal component is NeuGc $GM_2$. The acidic glycolipid derived from bovine red cell membrane which is spotted on the lane f is a compound which is assumed to include N-glycolyl type sialic acid. As seen from the results shown in Fig. 3, the supernatant of the culture medium of A 8 reacts only with NeuGc $GM_2$ (lane c) and that present in the acidic glycolipid of the mouse liver (lane e).

Then Fig. 3 will be explained below. In the following description, if there are a plurality of lines, they are listed in order from the top.

(1) Lane a: asialo $GM_2$, asialo $GM_1$, NeuAc $GM_1$;

(2) Lane b: NeuAc $GM_3$, NeuAc $GM_2$;

(3) Lane c: NeuGc $GM_3$, NeuGc $GM_2$;

(4) Lane d: CDH (upper two lines), CTH, paragloboside, $GD_{1a}$,. $GD_{1b}$;

(5) Lane e: the acidic glycolipid of the liver of BALB/c mice;

(6) Lane f: the acidic glycolipid of the bovine red cell membrane.

Thus, it is clear that the supernatant of the culture medium of A 8 specifically reacts with NeuGc $GM_2$, from the results plotted in Figs. 2 and 3.

It is also found that the hybridoma according to the present invention produces a monoclonal antibody which specifically recognizes N-glycolyl type $GM_2$.

## Claims

1. A monoclonal antibody specifically recognizing N-glycolyl type $GM_2$.

2. The monoclonal antibody according to claim 1 which, as determined by the Ouchterlony technique, belongs to the IgM class.

3. A hybridoma producing a monoclonal antibody according to claim 1 or 2.

4. The hybridoma according to claim 3 which is obtainable by fusing a B cell derived from the spleen of a BALB/c mouse with the BALB/c mice derived myeloma cell Sp 2/0.

5. The hybridoma according to claim 3 or 4 which is the hybridoma ATCC HB 9816.

6. A method for establishing a hybridoma according to any one of claims 3 to 5 comprising the fusion of (A) B cells obtained by immunizing an animal from which the thymus was removed when it was a neonate using a synthetic N-glycolyl type $GM_3$ as an immunogen, and (B) myeloma cells.

7. The method according to claim 6 wherein the animal is a BALB/c mouse from which the thymus was removed when it was a neonate.

8. The method according to claim 6 wherein the animal is an NTX mouse from which the thymus was removed when it was a neonate.

9. The method according to any one of claims 6 to 8 wherein the myeloma cell is derived from BALB/c mice.

10. The method according to claim 9 wherein the myeloma cell is the BALB/c mouse derived myeloma cell Sp 2/0.

11. The method according to any one of claims 6 to 10 wherein the immunization of the NTX mice is performed by intraperitoneally injecting the synthetic N-glycolyl type $GM_3$.

12. The method according to any one of claims 6 to 11 wherein the synthetic N-glycolyl type $GM_3$ is intraperitoneally injected together with an adjuvant.

13. The method according to any one of claims 6 to 12 wherein the adjuvant is killed Salmonella minesota in the form of a solution in phosphate-buffered saline.

14. The method according to any one of claims 6 to 13 wherein polyethylene glycol is used in the cell fusion as an agent for cell fusion.

15. The method according to any one of claims 6 to 14 wherein the fused cells are cultured after the cell fusion in HAT culture medium to separate hybridoma cells from the myeloma cells which remained unfused.

16. The method according to any one of claims 6 to 15 wherein the hybridoma is cloned by the methyl cellulose technique, soft agarose technique or limiting dilution technique.

# FIG.1

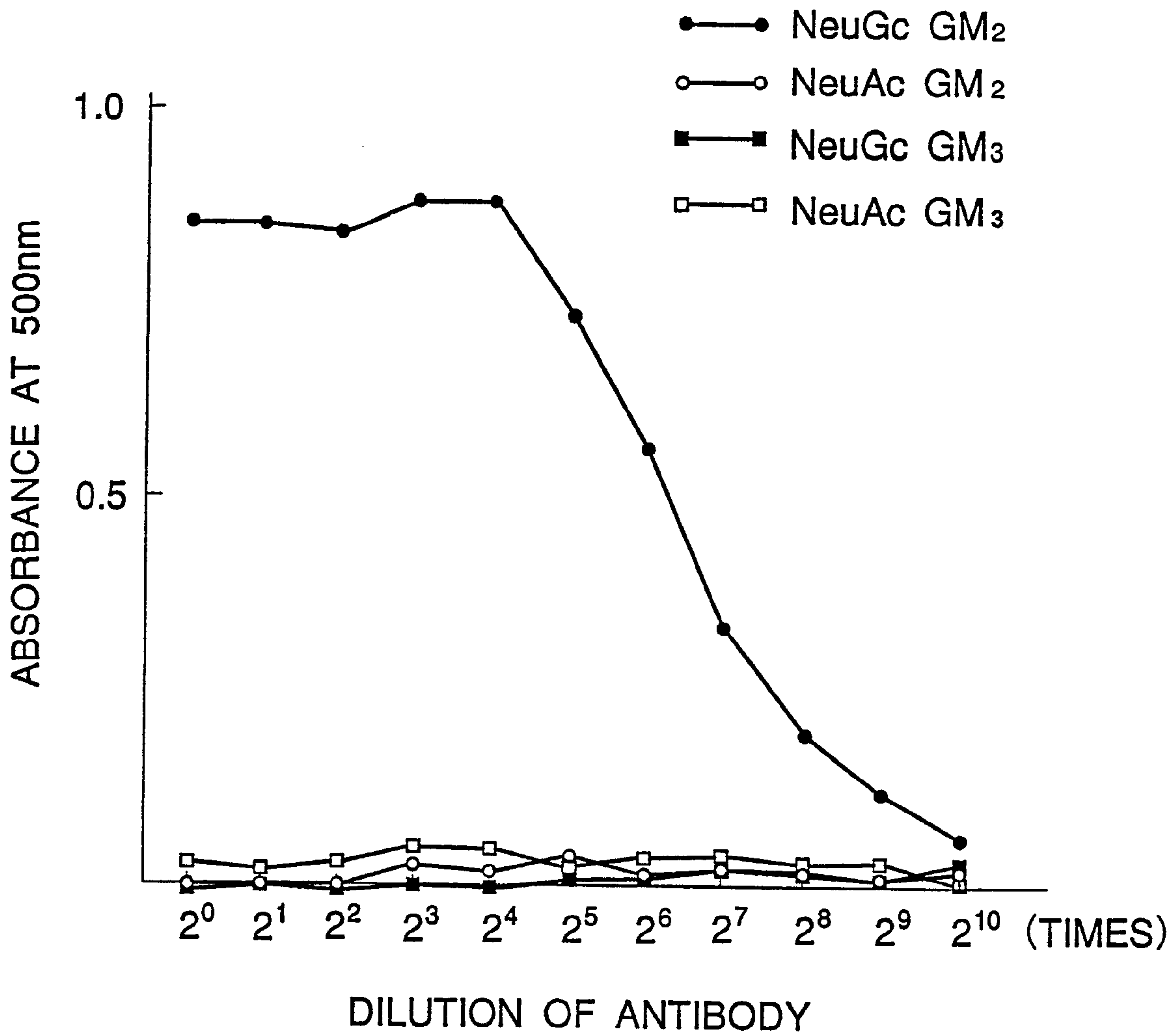
NeuGc GM2
NeuAc GM2
NeuGc GM3
NeuAc GM3
1.0
0.5
ABSORBANCE AT 500nm
2^0 2^1 2^2 2^3 2^4 2^5 2^6 2^7 2^8 2^9 2^10 (TIMES)
DILUTION OF ANTIBODY

# FIG.2

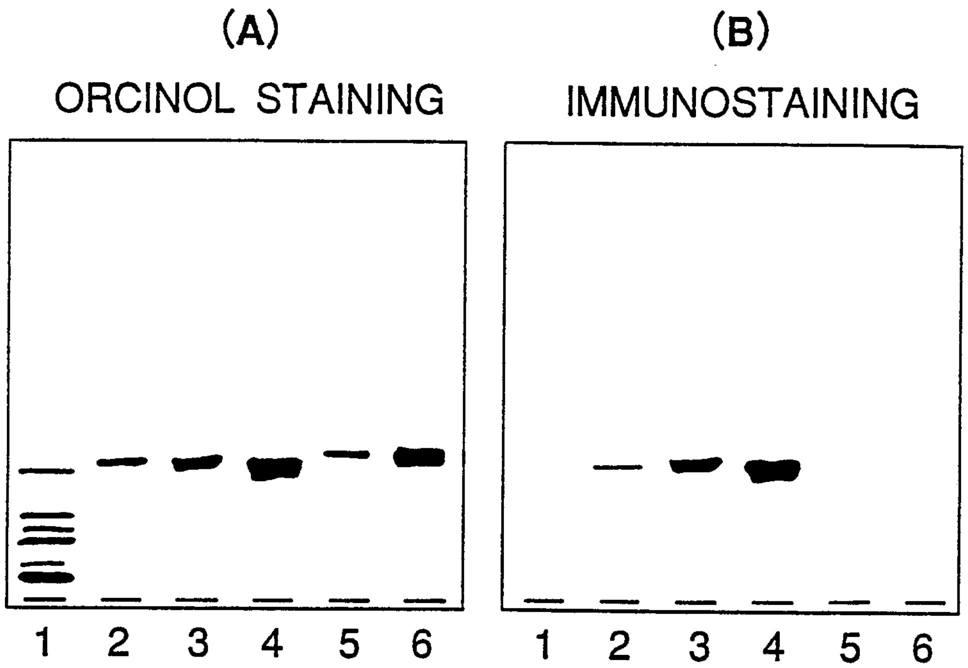
(A)
ORCINOL STAINING
(B)
IMMUNOSTAINING
1 2 3 4 5 6
1 2 3 4 5 6

# FIG.3

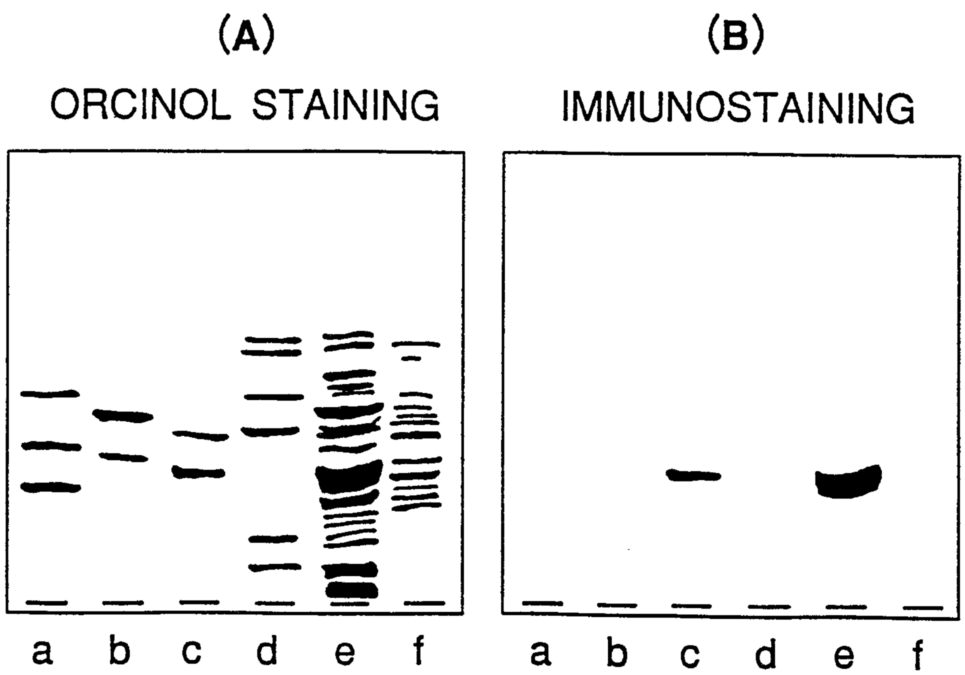
(A)
ORCINOL STAINING
(B)
IMMUNOSTAINING
a b c d e f
a b c d e f

European Patent Office

# EUROPEAN SEARCH REPORT

Application Number

EP 89 12 1138

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CANCER RESEARCH, vol. 48, no. 21, 1st November 1988, pages 6154-6160; M. MIYAKE et al.: "Generation of Two Murine Monoclonal Antibodies That Can Discriminate N-Glycolyl and N-Acetyl Neuraminic Acid Residues of Gm2 Gangliosides" * Page 6154, abstract * | 1-16 | C 12 P 21/08<br>C 12 N 5/24 |
| Y | PATENT ABSTRACTS OF JAPAN, vol. 13, no. 84, (C-572), 27th February 1989; & JP-A-63 270 699 (KONICA) * The whole abstract * | 1-16 | |
| Y | CANCER RESEARCH, vol. 46, no. 8, August 1986, pages 4116-4120; E.J. NATOLI et al.: "A murine Monoclonal Antibody Detecting N-Acetyl- and N-Glycolyl-Gm2: Characterization of Cell Surface Reactivity" * Page 4116, abstract * | 1-16 | |
| | | | TECHNICAL FIELDS SEARCHED (Int. Cl.5) |
| | | | C 12 P |

The present search report has been drawn up for all claims

| Place of search | Date of completion of the search | Examiner |
|---|---|---|
| THE HAGUE | 13-02-1990 | TURMO Y BLANCO C.E. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document